# FASCICULE DE BREVET EUROPEEN

(11) **EP 0 829 256 B1**
(45) Date de publication et mention de la délivrance du brevet: **04.06.2003**
(21) Numéro de dépôt: 97401968.9
(22) Date de dépôt: 21.08.1997
(51) Int. Cl.: A61K 7/06

(54) **Utilisation d'un agoniste des récepteurs des rétinoides de type RXR pour stimuler ou induire la pousse des cheveux et/ou stopper leur chute**
Verwendung von RXR-Retinoidrezeptor-Agonisten zum Stimulieren oder Induzieren des Haarwuchses und/oder zum Vermindern des Haarausfalles
Use of RXR retinoid receptor agonists to stimulate or induce hair growth and/or stop their loss

(30) Priorité: 09.09.1996 FR 9610968
(43) Date de publication de la demande: 18.03.1998
(73) Titulaire: L'OREAL, 75008 Paris (FR)
(72) Inventeur: Billoni, Nelly, 95760 Valmondois (FR); Mahe, Yann, 91390 Morsang-Sur-Orge (FR); Bernard, Bruno A., 92200 Neuilly S/Seine (FR)
(74) Mandataire: Tezier Herman, Béatrice

(56) Documents cités:
- EP-A- 0 253 393
- EP-A- 0 514 264
- EP-A- 0 694 301
- EP-A- 0 749 752
- WO-A-94/17796
- DE-A- 3 903 992

## Description

La présente invention concerne l'utilisation par voie topique d'une quantité efficace d'au moins un composé agoniste des récepteurs des rétinoides de type RXR, dans une composition destinée à induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Cependant différentes causes peuvent entraîner une perte importante, temporaire ou définitive, des cheveux. L'alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux. Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Il reste, d'une manière générale, qu'il serait intéressant et utile de pouvoir disposer de composés actifs autres que ceux déjà connus, potentiellement plus actifs et/ou moins toxiques.

Ce but et d'autres sont atteints par la présente invention qui concerne l'utilisation à titre de principe actif, dans un milieu physiologiquement acceptable, dans une composition cosmétique ou pour la préparation d'une composition pharmaceutique, d'une quantité efficace d'au moins un composé répondant à la formule générale (l) 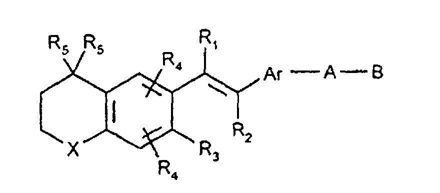 dans laquelle
Ar représente l'un des radicaux suivants 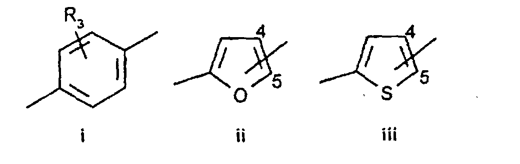 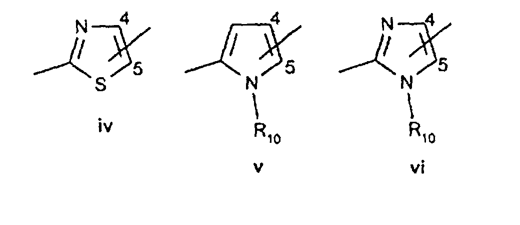 dans lesquels les hétérocycles pentagonaux sont substitués en position 4 ou 5 ;
A représente -(CH₂)ₙ avec n ayant une valeur de 0 à 5 ;
B représente un radical -COOH, ou un radical -COOR₇, -CONR₈R₉, -CH₂OH, -CH₂OR₁₀, -CH₂OCOR₁₀, -CHO, -CH(OR₁₁)₂, -CHOR₁₂O, -COR₆, -CR₆(OR₁₁)₂, -CR₆OR₁₂O;
X représente un atome d'oxygène, le radical -C-(R₅)₂, le radical S(O)ₘ avec m pouvant prendre les valeurs 0, 1 ou 2 ;
R₁ représente un atome d'halogène ou un radical alkyle ayant de 1 à 6 atomes de carbone ;
R₂ représente un atome d'hydrogène ou un radical alkyle ayant de 1 à 6 atomes de carbone ou un atome d'halogène ;
R₃ représente indépendamment ou simultanément un radical alkyle ayant de 1 à 6 atomes de carbone, un atome d'halogène, ou un radical -OR₁₀, -SR₁₀, -OCOR₁₀, -SCOR₁₀, -NH₂, -NHR₁₀, -N(R₁₀)₂ -NHCOR₁₀ ou -NR₁₀COR₁₀
R₄, indépendamment ou simultanément, représente un atome d'hydrogène, un radical alkyle ayant de 1 à 6 atomes de carbone, un atome d'halogène, un radical alkoxy ayant de 1 à 6 atomes de carbone ou un radical thioalkoxy ayant de 1 à 6 atomes de carbone ;
R₅, indépendamment ou simultanément, représente un atome d'hydrogène ou un radical alkyle ayant de 1 à 6 atomes de carbone ;
R₆ représente un radical alkyle ayant de 1 à 6 atomes de carbone , cycloalkyle ayant de 3 à 6 atomes de carbone ou alkenyle ayant de 2 à 6 atomes de carbone;
R₇ représente un radical alkyle ayant de 1 à 10 atomes de carbone, un radical cycloalkyle ayant de 5 à 10 atomes de carbone, ou radical phényle ou alkylphényle inférieur ;
R₈ et R₉ indépendamment ou simultanément représente un atome d'hydrogène, un radical alkyle ayant de 1 à 10 atomes de carbone, un radical cycloalkyle ayant de 5 à 10 atomes de carbone, un radical phényle ou un radical alkylphényle inférieur ;
R₁₀ représente indépendamment ou simultanément un radical alkyle ayant de 1 à 10 atomes de carbone, un radical phényle, ou un radical alkylphényle inférieur ;
R₁₁ représente un radical alkyle ayant de 1 à 6 atomes de carbone ;
R₁₂ représente un radical alkyle divalent ayant de 2 à 5 atomes de carbone ;
ses isomères optiques et/ou géométriques, ses formes acylées ou encore ses sels pharmaceutiquement acceptables pris seuls ou en mélange en toutes proportions ; ledit composé et/ou lesdites compositions étant administrés par voie topique, et destinés à induire et/ou stimuler la croissance des cheveux et/ou freiner leur chute.

Ces composés présentent des activités remarquables qui justifient leur utilisation comme principe actif pour induire et/ou stimuler la croissance des cheveux et/ou freiner leur chute.

A la connaissance de la demanderesse il n'a jamais été proposé dans l'art antérieur l'utilisation de tels composés pour lutter contre la chute des cheveux.

Les composés de formule I sont notamment décrits dans la demande de brevet WO-A-94/17796 pour leur propriété agoniste des récepteurs des rétinoides de types RXR.

Selon une forme de réalisation particulière de l'invention, l'atome d'halogène peut être un atome de chlore, de brome, de fluor ou d'iode, préférentiellement un atome de chlore, de brome ou d'iode.

Selon une forme de réalisation de l'invention R₁ représente un radical alkyle ayant de 1 à 6 atomes de carbone, et encore plus préférentiellement R₁ est un radical méthyle.

Selon une forme de réalisation de l'invention R₂ représente un atome d'hydrogène ou un radical alkyle ayant de 1 à 6 atomes de carbone et encore plus préférentiellement R₂ est un atome d'hydrogène.

Selon une autre forme de réalisation de l'invention R₃ représente un radical alkyle ayant de 1 à 6 atomes de carbone, et encore plus préférentiellement R₃ est un radical méthyle.

Selon une autre forme de réalisation de l'invention R₄ représente un atome d'hydrogène ou un radical alkyle ayant de 1 à 6 atomes de carbone et encore plus préférentiellement R₄ est un atome d'hydrogène.

Selon une autre forme de réalisation de l'invention R₅ représente un radical alkyle ayant de 1 à 6 atomes de carbone, et encore plus préférentiellement R₅ est un radical méthyle.

Selon une autre forme de réalisation de l'invention -A-B- représente un groupe -(CH₂)n-COOR₇, ou un groupe -(CH₂)ₙ-CONR₈R₉, (R₇, R₈ et R₉ étant définis comme précédement) et préférentiellement n est égal à zéro et B est un radical - COOR₇.

Préférentiellement, le composé utilisé selon l'invention est choisi parmi
l'acide 2-[(E)-2-(3,5,5,8,8-pentaméthyl-5,6,7,8-tétrahydro-2-naphtyl)-propen-1-yl]-4-thiophenecarboxylique ;
l'acide 2-[(E)-2-(3,5,5,8,8-pentaméthyl-5,6,7,8-tétrahydro-2-naphtyl)-propen-1-yl]-5-thiophenecarboxylique ;
l'acide 2-[(E)-2-(5,8,7,8-tétrahydro-3,5,5,8,8-pentaméthylnaphtalen-2-yl)-propen-1-yl]-4- furannecarboxylique ;
l'acide 4-[(E)-2-(3,5,5,8,8-pentaméthyl-5,6,7,8-tétrahydro-2-naphtyl)-propen-1-yl]-benzoïque ;
l'acide 2-[(E)-2-(4,4,7-triméthyl-6-thiochromanyl)-propen-1-yl]-4-thiophenecarboxylique ;
le 2-[(E)-2-(3,5,5,8,8-pentaméthyl-5,6,7,8-tétrahydro-2-naphtyl)-propen-1-yl]-4-thiophene carboxylate d'éthyle ;
l'acide 2-[(E)-2-(3,5,5,8,8-pentaméthyl-5,6,7,8-tétrahydro-2-naphtyl)-propen-1-yl]-4-thiazolecarboxylique ;
l'acide 2-[(E)-2-(3,5,5,8,8-pentaméthyl-5,6,7,8-tétrahydro-2-naphtyl)-propen-1-yl]-5-imidazolecarboxylique ;

Encore plus préférentiellement, le composé utilisé selon l'invention est l'acide 2-[(E)-2-(3,5,5,8,8-pentaméthyl-5,6,7,8-tétrahydro-2-naphtyl)-propen-1-yl]-4-thiophenecarboxylique .

Ces composés peuvent être utilisés seuls ou en mélange.

La quantité efficace de composé à utiliser correspond bien entendu à la quantité nécessaire pour obtenir le résultat désiré. L'homme du métier est donc en mesure d'évaluer cette quantité efficace qui dépend de la nature du composé utilisé et de la personne ainsi traitée. Pour donner un ordre de grandeur, dans les compositions selon l'invention le composé représente présent à une concentration comprise entre 0,001% et 10% en poids par rapport au poids total de la composition et de préférence comprise entre 0,01% et 1%.

Le milieu physiologiquement acceptable dans lequel l'actif est utilisé selon l'invention représente anhydre ou aqueux. On entend par milieu anhydre, un milieu solvant contenant moins de 1% d'eau. Ce milieu représente constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 6 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine cosmétique ou pharmaceutique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Il est possible aussi d'utiliser en association avec l'actif des composés améliorant encore l'activité sur la repousse et/ou sur le freinage de la chute des cheveux, et ayant déjà été décrits pour cette activité.

Parmi ces derniers composés, on peut plus particulièrement citer à titre non limitatif
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans les brevets US 4 139 619 et US 4 596 812 ;
- les agents favorisant la repousse des cheveux comme ceux décrits par la demanderesse dans la demande de brevet européen publiée sous le numéro 0648488;
- les agents antibactériens tels que les macrolides, les pyranosides et les tétracyclines, et notamment l'Erythromycine ;
- les agents antagonistes de calcium, comme la Cinnarizine, le Diltiazem, la Nimodipine et la Nifedipine ;
- des hormones, telles que l'estriol ou des analogues, ou la thyroxine et ses sels ;
- des agents anti-inflammatoires stéroïdiens, tels que les corticostéroïdes (par exemple : l'hydrocortisone) ;
- des agents antiandrogènes, tels que l'oxendolone, la spironolactone, le diéthylstilbestrol et la flutamide
- des inhibiteurs stéroïdiens ou non stéroïdiens des 5-α-réductases tels que le finastéride
- des agonistes potassiques tels que la cromakalim et le nicorandil.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un radical alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétrayénoïque et eicosatriyénoïque ou leurs esters et amides, la vitamine D et ses dérivés, des extraits d'origine végétale ou bactérienne.

On peut également envisager que la composition comprenant au moins un composé tel que défini précédement soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition pharmaceutique selon l'invention est administrée par voie topique.

La composition cosmétique selon l'invention est à appliquer sur les zones alopéciques du cuir chevelu et des cheveux d'un individu, et est éventuellement laissée en contact plusieurs heures et est éventuellement à rincer. On peut, par exemple, appliquer la composition contenant une quantité efficace d'au moins un composé tel que défini précédement, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, caractérisé par le fait qu'il consiste à appliquer sur les cheveux et/ou le cuir chevelu, une composition cosmétique comprenant une quantité efficace d'au moins composé tel que défini précédement, à laisser celle-ci en contact avec les cheveux et/ou le cuir chevelu, et éventuellement à rincer.

Le procédé de traitement présente les caractéristiques d'un procédé cosmétique dans la mesure où il permet d'améliorer l'esthétique des cheveux en leur donnant une plus grande vigueur et un aspect amélioré.

On va maintenant donner à titre d'illustration des exemples qui ne sauraient limiter en aucune façon la portée de l'invention.

### Exemple 1 : Mesure de l'effet de l'acide (E)-2-[(3,5,5,8,8-pentaméthyl-5,6,7,8-tétrahydro-2-naphtyl)-propen-1-yl]-4- thiophenecarboxylique sur l'allongement et la survie du follicule pileux maintenu in vitro.

Des follicules pileux viables *in vitro* sont préparés selon la technique décrite dans la demande de brevet n° 95-08465, déposée en France le 12 juillet 1995 par la demanderesse.

A partir d'une biopsie de scalp, une bande de cuir chevelu assez fine est isolée à l'aide d'un scalpel. Avec une micropince, le tissu adipeux entourant les follicules est éliminé à l'aide de micropinces en évitant d'endommager le bulbe pilaire. Sous un microscope, le follicule est coupé avec un scalpel pour le séparer de son environnement épidermique et dermique.
Les follicules sont ensuite mis en culture dans les puits de plaques 24 puits de type Costar, à raison d'un follicule par puits. Chaque puits contient 0,5 ml de milieu Williams E additionné de pénicilline et streptomycine à la concentration finale de 50 Ul/ml, de glutamine à de 2 mM, d'Insuline de boeuf à 0,01 mg/ml, et d'hydrocortisone à 0,04 µg/ml. Les follicules sont alors mesurés à l'aide d'un microscope muni d'un oculaire micrométrique.

Au bout de 24 heures les follicules sont de nouveau mesurés et ceux dont l'allongement est inférieur à 0,3 graduations du micromètre, correspondant à 0,16 mm, sont éliminés.

Les follicules conservés pour l'expérimentation sont alors mis en culture dans du milieu Williams E contenant ou ne contenant pas l'acide (E)-2-[(3,5,5,8,8-pentaméthyl-5,6,7,8-tétrahydro-2-naphtyl)-propen-1-yl]-4-thiophenecarboxylique .

Condition A : milieu williams E complet sans l'acide. Condition B : milieu Williams E complet + l'acide à la concentration de 10⁻⁸M.

### Résultats :

| *Condition A :* | | | | |
|---|---|---|---|---|
| Jours | allongement moyen | écart type | n | % survie |
| 0 | 0,00 | 0,00 | 11 | 100 |
| 3 | 0,96 | 0,14 | 11 1 | 100 |
| 4 | 1,22 | 0,20 | 11 1 | 100 |
| 5 | 1,48 | 0,24 | 11 1 | 100 |
| 6 | 1,64 | 0,28 | 11 1 | 100 |
| 7 | 1,85 | 0,32 | 11 1 | 100 |
| 10 | 2,72 | 0,34 | 8 | 73 |
| 11 1 | 2,97 | 0,43 | 8 | 73 |
| 12 | 3,20 | 0,49 | 8 | 73 |
| 13 | 3,47 | 0,58 | 8 | 73 |
| 14 | 3,76 | 0,62 | 8 | 73 |
| 19 | 4,89 | 0,99 | 8 | 73 |
| 21 | 5,55 | 0;95 | 8 | 73 |
| 26 | 5,75 | 0,88 | 8 | 73 |
| 27 | nd | - | 5 | 45 |
| 28 | nd | - | 4 | 36 |
| 31 | nd | - | 3 | 27 |
| 34 | nd | - | 3 | 27 |
| 35 | nd | - | 0 | 0 |

| Condition B : | | | | |
|---|---|---|---|---|
| Jours | allongement moyen | écart type | n | % survie |
| 0 | 0,00 | 0,00 | 12 | 100 |
| 3 | 1,18 | 0,07 | 12 | 100 |
| 4 | 1,47 | 0,08 | 12 | 100 |
| 5 | 1,75 | 0,13 | 12 | 100 |
| 6 | 2,04 | 0,18 | 12 | 100 |
| 7 | 2,29 | 0,26 | 12 | 100 |
| 10 | 3,30 | 0,42 | 11 | 92 |
| 11 1 | 3,60 | 0,46 | 11 1 | 92 |
| 12 | 4,06 | 0,29 | 10 | 83 |
| 13 | 4,53 | 0,41 | 10 | 83 |
| 14 | 4,85 | 0,44 | 10 | 83 |
| 19 | 6,80 | 0,36 | 9 | 75 |
| 21 | 7,54 | 0,33 | 9 | 75 |
| 26 | nd | - | 8 | 67 |
| 27 | nd | - | 8 | 67 |
| 28 | nd | - | 8 | 67 |
| 31 | nd | - | 7 | 58 |
| 34 | nd | - | 4 | 33 |
| 35 | nd | - | 0 | 0 |

Ces résultats montrent que l'acide (E)-2-[(3,5,5,8,8-pentaméthyl-5,6,7,8-tétrahydro-2-naphtyl)-propen-1 -yl]-4-thiophenecarboxylique favorise l'allongement du follicule pileux en culture et augmente son temps de survie. L'augmentation du temps de survie correspond à une augmentation de la durée de la phase du cycle dans laquelle se trouve le cheveu et permet ainsi d'en retarder la chute.

### Exemple 2 :

Exemples de compositions selon l'invention. Ces compositions sont obtenues par les techniques habituelles couramment utilisées en cosmétique ou en pharmacie.

Lotion antichute:
- Composé de formule 1 0,20 g
- Propylène glycol 10,00 g
- Alcool isopropylique qsp 100,00 g

On applique 1 ml de cette lotion sur le cuir chevelu, à la fréquence de une à deux fois par jour.

Lotion antichute:
- Composé de formule 1 0,01 g
- Propylène glycol 30,00 g
- Alcool éthylique 40,05 g
- Eau qsp 100,00 g

On applique cette lotion sur le cuir chevelu, une à deux fois par jour, à raison d'1 ml par application.

Lotion antichute épaissie :
- Composé de formule 1 0,20 g
- Kawaïne 2,00 g
- Hydroxypropylcellulose vendue par la société Hercules sous la dénomination Klucel G 3,50 g
- Alcool éthylique qsp 100,00 g

On applique cette lotion épaissie sur le cuir chevelu, une à deux fois par jour, à raison d'1 ml par application.

lotion antichute :
- Composé de formule 1 0,05 g
- Monométhyléther de propylèneglycol vendu sous la dénomination Dowanol PM par la société Dow Chemical 20,00 g
- Hydroxypropylcellulose vendue par la société Herculès sous la dénomination Klucel G 3,00 g
- Alcool éthylique 40,00 g
- Eau qsp 100,00 g

On applique cette lotion épaissie sur le cuir chevelu, une à deux fois par jour, à raison d'1 ml par application.

## Revendications

1. Utilisation, dans une composition cosmétique ou pour la préparation d'une composition pharmaceutique, d'une quantité efficace d'au moins d'au moins un composé répondant à la formule générale (I) 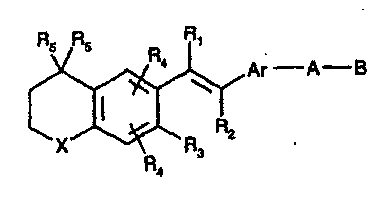 dans laquelle
Ar représente l'un **des** radicaux suivants 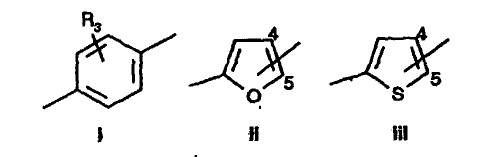 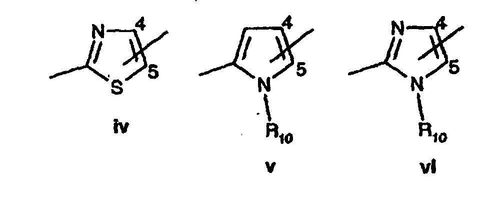 dans lesquels les hétérocycles pentagonaux sont substitués en position 4 ou 5 ;
A représente -(CH₂)ₙ avec n ayant une valeur de 0 à 5 ;
B représente un radical -COOH, ou un radical -COOR₇, -CONR₈R₉, -CH₂OH, -CH₂OR₁₀, -CH₂OCOR₁₀, -CHO, -CH(OR₁₁)₂, -CHOR₁₂O, -COR₆, -CR₆(OR₁₁)_{2,} -CR₆OR₁₂O;
X représente un atome d'oxygène, le radical -C-(R₅)₂, le radical S(O)ₘ avec m pouvant prendre les valeurs 0, 1 ou 2 ;
R₁ représente un atome d'halogène ou un radical alkyle ayant de 1 à 6 atomes de carbone ;
R₂ représente un atome d'hydrogène ou un radical alkyle ayant de 1 à 6 atomes de carbone ou un atome d'halogène ;
R₃ représente indépendamment ou simultanément un radical alkyle ayant de 1 à 6 atomes de carbone, un atome d'halogène, ou un radical -OR₁₀, -SR₁₀, -OCOR₁₀, -SCOR₁₀, -NH₂, -NHR₁₀, -N(R₁₀)₂ -NHCOR₁₀ ou -NR₁₀COR₁₀
R₄, indépendamment ou simultanément, représente un atome d'hydrogène, un radical alkyle ayant de 1 à 6 atomes de carbone, un atome d'halogène, un radical alkoxy ayant de 1 à 6 atomes de carbone ou un radical thioalkoxy ayant de 1 à 6 atomes de carbone ;
R₅, indépendamment ou simultanément, représente un atome d'hydrogène ou un radical alkyle ayant de 1 à 6 atomes de carbone ;
R₆ représente un radical alkyle ayant de 1 à 6 atomes de carbone , cycloalkyle ayant de 3 à, 6 atomes de carbone ou alkenyle ayant de 2 à 6 atomes de carbone ;
R₇ représente un radical alkyle ayant de 1 à 10 atomes de carbone, un radical cycloalkyle ayant de 5 à 10 atomes de carbone, ou radical phényle ou alkylphényle inférieur;
R₈ et R₉ indépendamment ou simultanément représente un atome d'hydrogène, un radical alkyle ayant de 1 à 10 atomes de carbone, un radical cycloalkyle ayant de 5 à 10 atomes de carbone, un radical phényle ou un radical alkylphényle inférieur ;
R₁₀ représente indépendamment ou simultanément un radical alkyle ayant de 1 à 10 atomes de carbone, un radical phényle, ou un radical alkylphényle inférieur ;
R₁₁ représente un radical alkyle ayant de 1 à 6 atomes de carbone ;
R₁₂ représente un radical alkyle divalent ayant de 2 à 5 atomes de carbone ;
ses isomères optiques et/ou géométriques, ses formes acylées ou encore ses sels pharmaceutiquement acceptables pris seuls ou en mélange en toutes proportions ;
ledit composé et/ou lesdites compositions étant administrés par voie topique, et destinés à induire et/ou stimuler la croissance des cheveux et/ou freiner leur chute.

2. Utilisation selon la revendication précédente, **caractérisée par le fait que** l'atome d'halogène est choisi parmi le fluor, le chlore, le brome ou l'iode.

3. Utilisation selon l'une quelconque des revendications précédentes, **caractérisée par le fait que** R₁ est un radical alkyle ayant de 1 à 6 atomes de carbone.

4. Utilisation selon l'une quelconque des revendications précédentes, **caractérisée par le fait que** R₂ est un atome d'hydrogène ou un radical alkyle ayant de 1 à 6 atomes de carbone.

5. Utilisation selon l'une quelconque des revendications précédentes, **caractérisée par le fait que** R₃ est un radical alkyle ayant de 1 à 6 atomes de carbone.

6. Utilisation selon l'une quelconque des revendications précédentes, **caractérisée par le fait que** R₄ est un atome d'hydrogène ou un radical alkyle ayant de 1 à 6 atomes de carbone.

7. Utilisation selon l'une quelconque des revendications précédentes, **caractérisée par le fait que** R₅ est un radical alkyle ayant de 1 à 6 atomes de carbone.

8. Utilisation selon l'une quelconque des revendications précédentes, **caractérisée par le fait que** -A-B- peut être choisi parmi un groupe -(CH₂)ₙ-COOR₇, ou un groupe -(CH₂)ₙ-CONR₈R₉ et préférentiellement n est égal à zéro et B est un radical -COOR₇.

9. Utilisation selon l'une quelconque des revendications précédentes, **caractérisée par le fait que** le composé est choisi parmi
l'acide 2-[(E)-2-(3,5,5,8,8-pentaméthyl-5,6,7,8-tétrahydro-2-naphtyl)-propen-1-yl]-4-thiophenecarboxylique ;
l'acide 2-[(E)-2-(3,5,5,8,8-pentaméthyl-5,6,7,8-tétrahydro-2-naphtyl)-propen-1 -yl]-5-thiophenecarboxylique ;
l'acide 2-[(E)-2-(5,8,7,8-tétrahydro-3,5,5,8,8-pentaméthylnaphtalen-2-yl)-propen-1-yl]-4-furannecarboxylique ;
l'acide 4-[(E)-2-(3,5,6,8,8-pentaméthyl-5,6,7,8-tétrahydro-2-naphtyl)-propen-1-yl]-benzoïque ;
l'acide 2-[(E)-2-(4,4,7-triméthyl-6-thiochromanyl)-propen-1-yl]-4-thiophenecarboxylique;
le 2-[(E)-2-(3,5,5,8,8-pentaméthyl-5,6,7,8 tétrahydro-2-naphtyl)-propen-1-yl]-4-thiophene carboxylate d'éthyle;
l'acide 2-[(E)-2-(3,5,5,8,8-pentaméthyl-5,6,7,8-tétrahydro-2-naphtyl)-propen-1-yl]-. 4-thiazolecarboxylique ;
l'acide 2-[(E)-2-(3,5,5,8,8-pentaméthyl-5,6,7,8-tétrahydro-2-naphtyl)-propen-1-yl]-5-imidazolecarboxyiique ;

10. Utilisation selon l'une quelconque des revendications. précédentes, **caractérisée par le fait que** le composé présent est utilisé à une concentration comprise entre 0,001% et 10 %, en poids par rapport au poids total de la composition et de préférence comprise entre 0,1 % et 1 %

11. Procédé de traitement cosmétique des cheveux et/ou du cuir chevelu, **caractérisé par le fait qu'**il consiste à appliquer sur les cheveux et/ou le cuir chevelu, une composition cosmétique telle que définie dans l'une quelconque des revendications 1 à 10, à laisser celle-ci en contact avec les cheveux et/ou le cuir chevelu, et éventuellement à rincer.

## Patentansprüche

1. Verwendung mindestens einer Verbindung der allgemeinen Formel (I) in einer wirksamen Menge in einer kosmetischen Zusammensetzung oder zur Herstellung einer pharmazeutischen Zusammensetzung: 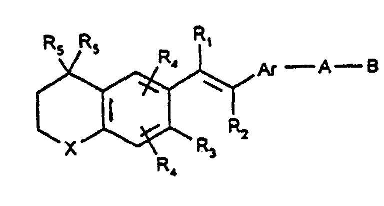 wobei in der Formel bedeuten:
- Ar eine der folgenden Gruppen: 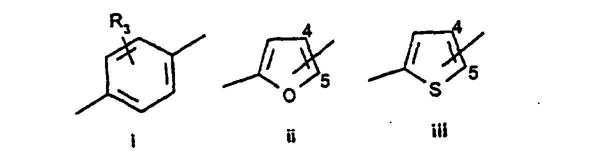 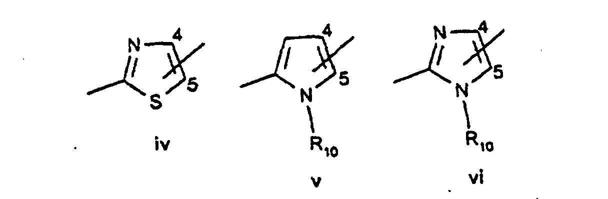
wobei die pentagonalen Heterocyclen in 4- oder 5-Stellung substituiert sind;
- A die Gruppe (CH₂)ₙ, wobei n einen Wert von 0 bis 5 hat;
- B -COOH, -COOR₇, -CONR₈R₉, -CH₂OH, -CH₂OR₁₀, -CH₂OCOR₁₀,
- CHO, -CH(OR₁₁)₂, -CHOR₁₂O, -COR₆, -CR₆(OR₁₁)₂, -CR₆OR₁₂O;
- X ein Sauerstoffatom, eine Gruppe -C-(R₅)₂ oder eine Gruppe S(O)m, wobei m die Werte 0, 1 oder 2 annehmen kann;
- R1 ein Halogenatom oder eine Alkylgruppe mit 1 bis 6 Kohlenstoffatomen;
- R₂ ein Wasserstoffatom, eine Alkylgruppe mit 1 bis 6 Kohlenstoffatomen oder ein Halogenatom;
- die Gruppen R₃ unabhängig voneinander oder gleichzeitig eine Alkylgruppe mit 1 bis 6 Kohlenstoffatomen, ein Halogenatom oder -OR₁₀, -SR₁₀, -OCOR₁₀, -SCOR₁₀, -NH₂, -NHR₁₀, -N(R₁₀)₂, -NHCOR₁₀ oder -NR₁₀COR₁₀;
- die Gruppen R₄ unabhängig voneinander oder gleichzeitig ein Wasserstoffatom, eine Alkylgruppe mit 1 bis 6 Kohlenstoffatomen, ein Halogenatom, eine Alkoxygruppe mit 1 bis 6 Kohlenstoffatomen oder eine Thioalkoxygruppe mit 1 bis 6 Kohlenstoffatomen;
- die Gruppen R₅ unabhängig voneinander oder gleichzeitig ein Wasserstoffatom oder eine Alkylgruppe mit 1 bis 6 Kohlenstoffatomen;
- R₆ eine Alkylgruppe mit 1 bis 6 Kohlenstoffatomen, eine Cyclöalkylgruppe mit 3 bis 6 Kohlenstoffatomen oder eine Alkenylgruppe mit 2 bis 6 Kohlenstoffatomen;
- R₇ eine Alkylgruppe mit 1 bis 10 Kohlenstoffatomen, eine Cycloalkylgruppe mit 5 bis 10 Kohlenstoffatomen, Phenyl oder eine niedere Alkylphenylgruppe;
- die Gruppen R₈ und R₉ unabhängig voneinander oder gleichzeitig ein Wasserstoffatom, eine Alkylgruppe mit 1 bis 10 Kohlenstoffatomen, eine Cycloalkylgruppe mit 5 bis 10 Kohlenstoffatomen, Phenyl oder eine niedere Alkylphenylgruppe;
- die Gruppen R₁₀ unabhängig voneinander oder gleichzeitig eine Alkylgruppe mit 1 bis 10 Kohlenstoffatomen, Phenyl oder eine niedere Alkylphenylgruppe;
- R₁₁ eine Alkylgruppe mit 1 bis 6 Kohlenstoffatomen;
- R₁₂ eine zweiwertige Alkylgruppe mit 2 bis 5 Kohlenstoffatomen;
und der optischen und/oder geometrischen Isomere dieser Verbindungen, ihrer acylierten Formen oder ihrer pharmazeutisch akzeptablen Salze einzeln oder im Gemisch in beliebigen Mengenverhältnissen;
wobei die Verbindung und/oder die Zusammensetzungen topisch verabreicht werden und dazu vorgesehen sind, das Wachstum der Haare zu induzieren und/oder zu stimulieren und/oder den Haarausfall zu verlangsamen.

2. Verwendung nach dem vorhergehenden Anspruch, **dadurch gekennzeichnet, dass** das Halogenatom unter Fluor, Chlor, Brom oder Iod ausgewählt ist.

3. Verwendung nach einem der vorhergehenden Ansprüche, **dadurch gekennzeichnet, dass** R₁ eine Alkylgruppe mit 1 bis 6 Kohlenstoffatomen bedeutet.

4. Verwendung nach einem der vorhergehenden Ansprüche, **dadurch gekennzeichnet, dass** R₂ Wasserstoff oder eine Alkylgruppe mit 1 bis 6 Kohlenstoffatomen bedeutet.

5. Verwendung nach einem der vorhergehenden Ansprüche, **dadurch gekennzeichnet, dass** R₃ eine Alkylgruppe mit 1 bis 6 Kohlenstoffatomen bedeutet.

6. Verwendung nach einem der vorhergehenden Ansprüche, **dadurch gekennzeichnet, dass** R₄ Wasserstoff oder eine Alkylgruppe mit 1 bis 6 Kohlenstoffatomen bedeutet.

7. Verwendung nach einem der vorhergehenden Ansprüche, **dadurch gekennzeichnet, dass** R₅ eine Alkylgruppe mit 1 bis 6 Kohlenstoffatomen bedeutet.

8. Verwendung nach einem der-vorhergehenden Ansprüche, **dadurch gekennzeichnet, dass** die Gruppe -A-B- unter -(CH₂)ₙ-COOR₇ oder - (CH₂₎ₙ₋CONR₈R₉ ausgewählt ist, wobei vorzugsweise n 0 ist und B die Gruppe -COOR₇ bedeutet.

9. Verwendung nach einem der vorhergehenden Ansprüche, **dadurch gekennzeichnet, dass** die Verbindung ausgewählt ist unter:
- 2-[(E)-2-(3,5,5,8,8-Pentamethyl-5,6,7,8-tetrahydro-2-naphthyl)-propen-1-yl]-4-thiophencarbonsäure;
- 2-[(E)-2-(3,5,5,8,8-Pentamethyl-5,6,7,8-tetrahydro-2-naphthyl)-propen-1-yl]- 5-thiophencarbonsäure;
- 2-[(E)-2-(5,6,7,8-Tetrahydro-3,5,5,8,8-pentamethyl-naphthalin-2 yl)-propen-1-yl]-4-furancarbonsäure;
- 4-[(E)-2-(3,5,5,8,8-Pentamethyl-5,6,7,8-tetrahydro-2-naphthyl)-propen-1-yl]-benzoesäure;
- 2-[(E)-2-(4,4,7-Trimethyl-6-thiochromanyl)-propen-1-yl]-4-thiophencarbonsäure;
- Ethyl-2-[(E)-2-(3,5,5,8,8-pentamethyl-5,6,7,8-tetrahydro-2-naphthyl)-propen-1-yl]-4-thiophencarboxylat;
- 2-[(E)-2-(3,5,5,8,8-Pentamethyl-5,6,7,8-tetrahydro-2-naphthyl)-propen- 1-yl]-4-thiazolcarbonsäure; und
- 2-[(E)-2-(3,5,5,8,8-Pentamethyl-5,6,7,8-tetrahydro-2-naphthyl)-propen-1-yl]-5-imidazolcarbonsäure.

10. Verwendung nach einem der vorhergehenden Ansprüche, **dadurch gekennzeichnet, dass** die Verbindung in einer Konzentration im Bereich von 0,001 bis 10 Gew.-%, bezogen auf das Gesamtgewicht der Zusammensetzung, und vorzugsweise in einer Menge von 0,01 bis 1 % vorliegt.

11. Verfahren zur kosmetischen Behandlung der Haare und/oder der Kopfhaut, **dadurch gekennzeichnet, dass** es darin besteht, auf die Haare und/oder die Kopfhaut eine kosmetische Zusammensetzung nach einem der Ansprüche 1 bis 10 aufzutragen, diese mit den Haaren und/oder der Kopfhaut in Kontakt zu belassen und gegebenenfalls zu spülen.

## Claims

1. Use in a cosmetic composition, or for the preparation of a pharmaceutical composition, of an effective amount of at least one compound corresponding to the general formula (I): 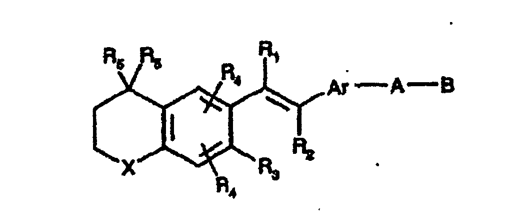 in which
Ar represents one of the following radicals: 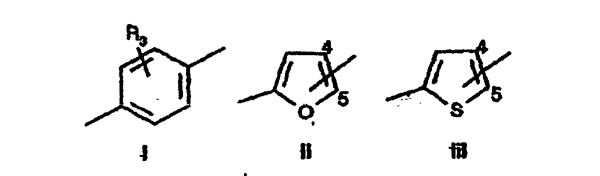 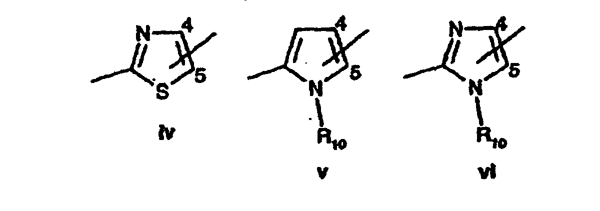 in which the pentagonal heterocycles are substituted in position 4 or 5;
A represents -(CH₂)ₙ with n having a value from 0 to 5; B represents a -COOH radical or a radical -COOR₇, - CONR₈R₉. -CH₂OH, -CH₂OR₁₀, -CH₂OCOR₁₀, -CHO, -CH(OR₁₁)₂, - CHOR₁₂O, -COR₆, -CR₆ (OR₁₁)₂, -CR₆OR₁₂O;
X represents an oxygen atom, the radical -C-(R₅)₂ or the radical S(O)m with m being able to take the values 0, 1 or 2;
R₁ represents a halogen atom or an alkyl radical having from 1 to 6 carbon atoms;
R₂ represents a hydrogen atom or an alkyl radical having from 1 to 6 carbon atoms or a halogen atom;
R₃ represents, independently or simultaneously, an alkyl radical having from 1 to 6 carbon atoms, a halogen atom or a radical -OR₁₀, -SR₁₀, -OCOR₁₀, -SCOR₁₀, -NH₂, -NHR₁₀, -N (R₁₀)₂, -NHCOR₁₀ or -NR₁₀COR₁₀;
R₄, independently or simultaneously, represents a hydrogen atom, an alkyl radical having from 1 to 6 carbon atoms, a halogen atom, an alkoxy radical having from 1 to 6 carbon atoms or a thioalkoxy radical having from 1 to 6 carbon atoms;
R₅, independently or simultaneously, represents a hydrogen atom or an alkyl radical having from 1 to 6 carbon atoms;
R₆ represents an alkyl radical having from 1 to 6 carbon atoms, a cycloalkyl radical having from 3 to 6 carbon atoms or an alkenyl radical having from 2 to 6 carbon atoms;
R₇ represents an alkyl radical having from 1 to 10 carbon atoms, a cycloalkyl radical having from 5 to 10 carbon atoms or a phenyl or lower alkylphenyl radical; R₈ and R₉, independently or simultaneously, represent a hydrogen atom, an alkyl radical having from 1 to 10 carbon atoms, a cycloalkyl radical having from 5 to 10 carbon atoms, a phenyl radical or a lower alkylphenyl radical;
R₁₀ represents, independently or simultaneously, an alkyl radical having from 1 to 10 carbon atoms, a phenyl radical or a lower alkylphenyl radical;
R₁₁ represents an alkyl radical having from 1 to 6 carbon atoms;
R₁₂ represents a divalent alkyl radical having from 2 to 5 carbon atoms;
its optical and/or geometrical isomers, its acyl forms or alternatively its pharmaceutically acceptable salts taken alone or as a mixture in all proportions;
the said compound and/or the said compositions being administered topically and intended to induce and/or stimulate hair growth and/or slow down hair loss.

2. Use according to the preceding claim, **characterized in that** the halogen atom is chosen from fluorine, chlorine, bromine or iodine.

3. Use according to either of the preceding claims, **characterized in that** R₁ is an alkyl radical having from 1 to 6 carbon atoms.

4. Use according to any one of the preceding claims, **characterized in that** R₂ is a hydrogen atom or an alkyl radical having from 1 to 6 carbon atoms.

5. Use according to any one of the preceding claims, **characterized in that** R₃ is an alkyl radical having from 1 to 6 carbon atoms.

6. Use according to any one of the preceding claims, **characterized in that** R₄ is a hydrogen atom or an alkyl radical having from 1 to 6 carbon atoms.

7. Use according to any one of the preceding claims, **characterized in that** R₅ is an alkyl radical having from 1 to 6 carbon atoms.

8. Use according to any one of the preceding claims, **characterized in that** -A-B- may be chosen from a group-(CH₂)ₙ-COOR₇, or a group - (CH₂) ₙ-CONR₈R₉ and, preferably, n is equal to zero and B is a radical -COOR₇.

9. Use according to any one of the preceding claims, **characterized in that** the compound is chosen from:
2-[(E)-2-(3,5,5,8,8-pentamethyl-5,6,7,8-tetrahydro-2-naphthyl)propen-1-yl]-4-thiophenecarboxylic acid;
2-[(E)-2-(3,5,5,8,8-pentamethyl-5,6,7,8-tetrahydro-2-naphthyl)propen-1-yl]-5-thiophenecarboxylic acid;
2-[(E)-2-(5,6,7,8-tetrahydro-3, 5,5,8,8-pentamethylnaphthalen-2-yl)propen-1-yl]-4-furancarboxylic acid;
4-[(E)-2-(3,5,5,8,8-pentamethyl-5,6,7,8-tetrahydro-2-naphthyl)propen-1-yl]benzoic acid;
2-[(E)-2-(4,4,7-trimethyl-6-thiochromanyl)propen-1-yl]-4-thiophenecarboxylic acid;
ethyl 2[(E)-2-(3,5,5,8,8-pentamethyl-5,6,7,8-tetrahydro-2-naphthyl)propen-1-yl]-4-thiophenecarboxylate;
2-[(E)-2-(3,5,5,8,8-pentamethyl-5,6,7,8-tetrahydro-2-naphthyl)propen-1-yl]-4-thiazolecarboxylic acid;
2-[(E)-2-(3,5,5,8,8-pentamethyl-5,6,7,8-tetrahydro-2-naphthyl)propen-1-yl]-5-imidazolecarboxylic acid.

10. Use according to any one of the preceding claims, **characterized in that** the compound present is used at a concentration of between 0.001% and 10% by weight relative to the total weight of the composition and preferably between 0.1% and 1%.

11. Process for the cosmetic treatment of the hair and/or the scalp, **characterized in that** it consists in applying a cosmetic composition as defined in any one of Claims 1 to 10 to the hair and/or the scalp, in leaving this composition in contact with the hair and/or the scalp and optionally in rinsing it out.
